# EUROPEAN PATENT APPLICATION

(11) **EP 2 159 036 A2**
(43) Date of publication of application: **03.03.2010**
(21) Application number: 09010034.8
(22) Date of filing: 04.08.2009
(51) Int. Cl.: B29C 65/02

(54) **Welding apparatus, tubular body with a covered tip end and welding method**

(30) Priority: 29.08.2008 JP 2008221248
(71) Applicant: Tyco Healthcare Group LP, Mansfield, MA 02048 (US)
(72) Inventor: Iwamizu, Keita, Fukuroi Shizuoka 437-0004 (JP)
(74) Representative: Olsson, Carl H.S.

(57) **Abstract**

The tip end of a tubular body is oriented towards an inner bottom face of a recess for holding a heating body, then the outer peripheral surface is oriented towards the inner peripheral surface of a heating body, and said tubular body is inserted into the heating body from the open side of the recess in the axial direction. This allows a film to be shaped from the tip end around the outer peripheral surface of the tubular body. The film and the heating body are heated in an overlapping state, and the film is welded to the outer peripheral surface of the tubular body.

## Description

### FIELD OF THE DISCLOSURE

The present disclosure relates to a welding apparatus, a tubular body with a covered tip end produced using the welding apparatus, and a welding method.

### BACKGROUND OF THE DISCLOSURE

Thin filters and sheets are conventionally fixed to the openings of cylindrical members by welding or bonding, and use is made of a welding apparatus. These apparatuses comprise an annular receiving jig and an ultrasonic horn shaped like a mortar in which an annular projection and a circular bottom part are formed. A filter joint which is a hard pipe is held in the receiving jig, with a mesh filter being fitted on the upper end face of the filter joint, and the ultrasonic horn is arranged over the filter, by means of which the filter is fixed to the filter joint. The ultrasonic horn is lowered to fuse part of the filter joint so that the filter is fixed to the upper face of the filter joint. In this process, the filter is cut by means of the annular projection.

However, with the method described above in which a conventional welding apparatus is employed, the filter is fixed to the end face of the filter joint and the outer peripheral part of the fixed portion thereof is cut, and therefore burring of the filter is likely to occur at the outer periphery of the end part of the filter joint. As a result, two-stage processing is required, and the filter is likely to become detached from the filter joint. That is, when an external force in the axial direction of the filter joint is applied to the filter so as to detach the filter from the end face of the filter joint, the filter is likely to become detached. Furthermore, if the filter joint is thin, the area of the end face is small, and therefore the force fixing the filter to the end face of the filter joint is weak.

The present disclosure has been devised in order to overcome such problems, and it aims to provide a welding apparatus, a tubular body with a covered tip end and a welding method with which it is possible to weld a covering material onto a tubular body regardless of the thickness of the tubular body, and also with which said covering material does not become readily detached from the tubular body.

### SUMMARY OF THE DISCLOSURE

The present disclosure relates to a welding apparatus that is provided with a holding stand having a recess for holding a heating body, a cylindrical heating body which is arranged inside the recess for holding a heating body in a state in which the outer peripheral surface is supported by the peripheral surface of the recess for holding a heating body, and heating means for heating the heating body, and in said welding apparatus a tubular body and a covering material are inserted into the heating body so that the tubular body is caught from the tip end opening at the outer periphery and covered by the covering material, and the covering material is welded to the outer periphery of the tubular body by the heat generated by the heating body which is heated by means of the operation of the heating means.
The present disclosure also relates to a method for producing a tubular body with a covered tip end wherein a covering material is welded to the outer periphery of the tubular body. The method comprises:
(a) inserting a tubular body and a covering material into a cylindrical heating body,
(b) catching the tubular body from the tip end opening, at the outer periphery of the tubular body,
(c) covering the tubular body with the covering material,and
(d) heating the cylindrical heating body to generate heat to weld the covering material to the outer periphery of the tubular body.
In another embodiment, the method for producing the tubular body with a covered tip end wherein the covering material is welded to the outer periphery of the tubular body, is carried out in a welding apparatus provided with a holding stand having a recess for holding a heating body, a cylindrical heating body which is arranged inside the recess for holding a heating body in a state in which the outer peripheral surface is supported by the peripheral surface of the recess for holding a heating body, and heating means for heating the body.
The present disclosure also relates to a tubular body with a covered tip end in which a covering material is welded to the outer periphery of the tubular body, when produced using the method of the present disclosure, or the welding apparatus of the present disclosure, and/or both the method and welding apparatus of the present disclosure.

### BRIEF DESCRIPTION OF THE DRAWINGS

Figure 1 is a view in cross section which has been cut away in the axial direction of the tubular body, showing a mode of embodiment of the welding device according to the present disclosure;
Figure 2 is a view showing the state before the tubular body is inserted into the heating body in the recess for holding a heating body;
Figure 3 is a view showing a state in which the tubular body has been removed from inside the heating body in the recess for holding a heating body, after the film has been welded;
Figure 4 is a partial schematic oblique view showing the curve-forming member together with the endoscope and endoscope sheath; and
Figure 5 is a partial schematic oblique view showing the tubular portion of the endoscope sheath for another tubular body.
Corresponding reference numerals indicate corresponding parts throughout the drawings, and herein the following reference numerals apply:
10...welding apparatus; 11...holding stand;12...heating body; 13...induction-heating coil; 14...recess for holding a heating body; 15...protrusion; 16, 36...covering material; 17...holding pin; 23...tubular body with a covered tip end; 23a, 33a...tubular body; 23b, 33b...window part corresponding to the tip end of an endoscope; 33...endoscope sheath.

### DETAILED DESCRIPTION OF THE DISCLOSURE

With the welding apparatus according to the present disclosure which is configured in this manner, the tubular body and the covering material are inserted into the heating body so that the tubular body is caught from the tip end opening at the outer periphery and covered by the covering material, and in this state the heating means are actuated so that the heating body is caused to generate heat. Accordingly, the covering material is welded to the outer periphery of the tubular body by the heat generated by the heating body. In this case, the welding of the covering material to the tubular body takes place at the outer periphery of the tubular body, and therefore the covering material can be reliably welded to the tubular body even if the tubular body is thin and has an extremely small area at its end face. Furthermore, the area of the welded portion can also be freely adjusted.

In addition, the covering material is welded to the outer periphery of the tubular body, and therefore the covering material is unlikely to become detached even if an external force in the axial direction of the tubular body is applied to the covering material. Moreover, the heating means used according to the present disclosure may be means for heating by induction heating, or means for heating from an external heat source such as a heater, or the like, and in this case the heating means consist of an induction-heating coil or a heat source such as a heater. Moreover, a film may be cited as the covering material.

Further structural features of the welding apparatus according to the present disclosure lie in the fact that a cylindrical protrusion which projects outwards is provided at the opening edge part of the recess for holding a heating body in the holding stand. This means that the portion of the covering material which is not welded to the tubular body is blocked by the cylindrical protrusion, making it unlikely to come into contact with the heating body, and therefore it is possible to prevent the portion of the covering material which is not welded to the tubular body from slackening and coming into contact with the heating body so that it is welded to the heating body. Furthermore, it is simple to apply tension to the covering material by bringing the covering material into contact with the protrusion, and this tension allows the covering material to be fusion-cut at the end of the heating body on the side in the opposite direction to the direction of insertion of the tubular body (the boundary between the welded portion and unwelded portion) at the same time as the covering material is welded, which means that no burring is generated at the end of the covering material, and there is no need for two-stage processing, and a pleasing external appearance can be obtained.

Further structural features of the welding apparatus according to the present disclosure lie in the fact that provision is made for a holding pin for insertion into the tubular body, the holding pin preventing deformation of the tubular body inside the tube by insertion of said holding pin into the tubular body. This means that the covering material can be welded to the tubular body with the holding pin inserted into the tubular body. Deformation of the tubular body inside the tube is avoided (support of the inner diameter) during welding so that it is possible to prevent wrinkling of the covering material and buckling of the tubular body. This is especially effective when the tubular body is thin.

Structural features of the tubular body with a covered tip end according to the present disclosure lie in the fact that the covering material is welded to the outer periphery thereof using the welding apparatus described above. This means that, as described above, the welding of the covering material to the tubular body takes place at the outer periphery of the tubular body, and therefore the covering material can be welded to the outer periphery of the tubular body regardless of the thickness of the tubular body, and furthermore the covering material can be made less likely to become detached from the tubular body.

Distinguishing features of the welding method according to the present disclosure lie in the fact that it comprises: a set-up step in which a tubular body and a covering material are inserted into a cylindrical heating body so that the tubular body is caught from the tip end opening at the outer periphery and covered by the covering material; and a welding step in which the heating body is heated so that the covering material is welded to the outer periphery of the tubular body by the heat generated by the heating body. This means that, as described above, the welding of the covering material to the tubular body takes place at the outer periphery of the tubular body, and therefore the covering material can be welded to the outer periphery of the tubular body to close off the opening of the tubular body, regardless of the thickness of the tubular body, and furthermore the covering material can be made less likely to become detached from the tubular body.

Further distinguishing features of the welding method according to the present disclosure lie in the fact that the tubular body is the tubular portion of an endoscope sheath into which an endoscope is inserted and the abovementioned covering material is a film, and the endoscope sheath is formed by closing off the tip end opening of the tubular body using the film. This means that it is possible to obtain an endoscope sheath in which the covering material is welded to the outer periphery and which is formed with a window part corresponding to the tip end of the endoscope, whereof the tip end opening is closed off by the film. Furthermore, if a tubular member for bending the endoscope is fitted to the tip end of the endoscope sheath, the covering material may be attached to said tubular member.

The welding method for producing a tubular body with a covered tip end wherein a covering material is welded to the outer periphery of the tubular body may be carried out, in one embodiment, in a welding apparatus provided with a holding stand having a recess for holding a heating body, a cylindrical heating body which is arranged inside the recess for holding a heating body in a state in which the outer peripheral surface of the heating body is supported by the peripheral surface of the recess for holding a heating body, and heating means for heating the heating body.
In another embodiment, the welding apparatus is provided with a cylindrical protrusion which projects outwards at the opening edge part of the recess for holding a heating body in the holding stand.
In another embodiment, the welding apparatus is provided with a holding pin for insertion into the tubular body, the holding pin preventing deformation of the tubular body inside the tube by insertion of said holding pin into the tubular body.
In another embodiment, the welding apparatus is provided with a holding pin for insertion into the tubular body, the holding pin preventing deformation of the tubular body inside the tube by insertion of said holding pin into the tubular body.

### DETAILED DESCRIPTION OF THE DRAWINGS

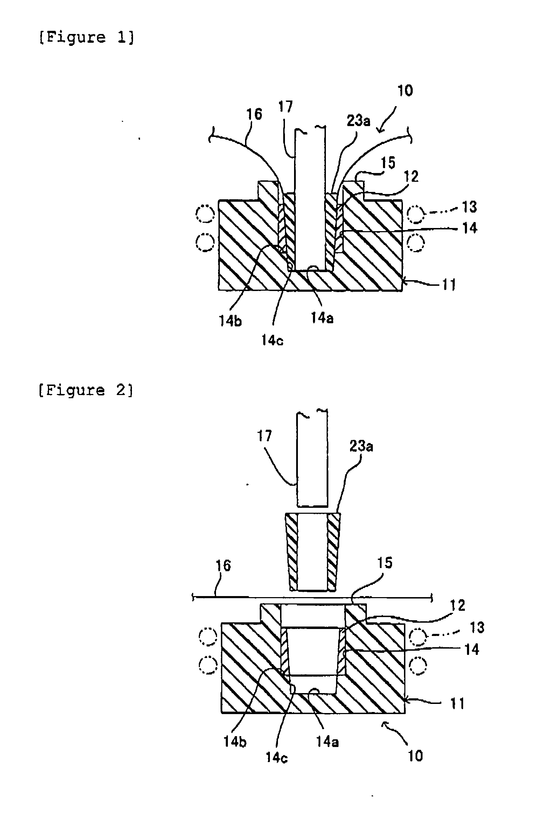

A mode of embodiment of the welding apparatus according to the present disclosure will be described with reference to Figures 1 to 3. As shown in Figure 1, a film welding apparatus 10 which serves as the welding apparatus according to the present invention comprises a holding stand 11, a heating body 12 which is arranged in the holding stand 11, and an induction-heating coil 13 (shown by the two-dash chain line) which serves as the heating means of the present invention and heats the heating body 12. The holding stand 11 consists of a non-heating body made of a fluororesin or a ceramic, and it is formed in the shape of a circular block in the form of a short column wherein the vertical direction is the axial direction. Furthermore, a recess 14 for holding a heating body which is open at the top is formed on the upper face of the holding stand 11.

This recess 14 for holding a heating body consists of a stepped recess which is circular in any transverse section in the axial direction, in which the upper side has a larger diameter than the lower side, and the diameter of the upper portion is set to be constant, with the diameter of the lower portion becoming steadily smaller moving downwards from the upper part. That is the recess 14 for holding a heating body comprises an inner bottom face 14a at the very bottom and a stepped part 14b lying at the boundary between the upper portion and the lower portion, and an inner peripheral surface 14c lying between the inner bottom face 14a and the stepped part 14b is formed as a tapering surface wherein the lower part is of smaller diameter than the upper part. A cylindrical protrusion 15 projecting upwards is formed at the opening edge part of the recess 14 for holding a heating body in the holding stand 11. The inner diameter of the protrusion 15 is the same as the diameter of the upper portion of the recess 14 for holding a heating body.

The heating body 12 has a cylindrical shape in which the vertical direction is the axial direction, and it is positioned at the upper portion of the recess 14 for holding a heating body. The outer diameter of the heating body 12 is the same in the axial direction (the vertical direction in Figure 1), and the inner diameter of the heating body 12 contracts from the top towards the bottom so that it has a tapering shape (the inner shape is that of a truncated cone). Furthermore, the inner peripheral surface of the heating body 12 consists of a curving surface which is contiguous with the inner peripheral surface 14c of the recess 14 for holding a heating body. The heating body 12 is made of a metal, and in the case of induction heating, it is preferably a stainless steel which demonstrates very efficient heat generation, such as SUS403, for example. If a heater or the like is used for the heating, the heating body is preferably copper which has high heat conduction.

The coil 13 is wound around the outer periphery of the holding stand 11, and the heating body 12 is heated to generate heat through induction heating by passing electrical current through the coil.

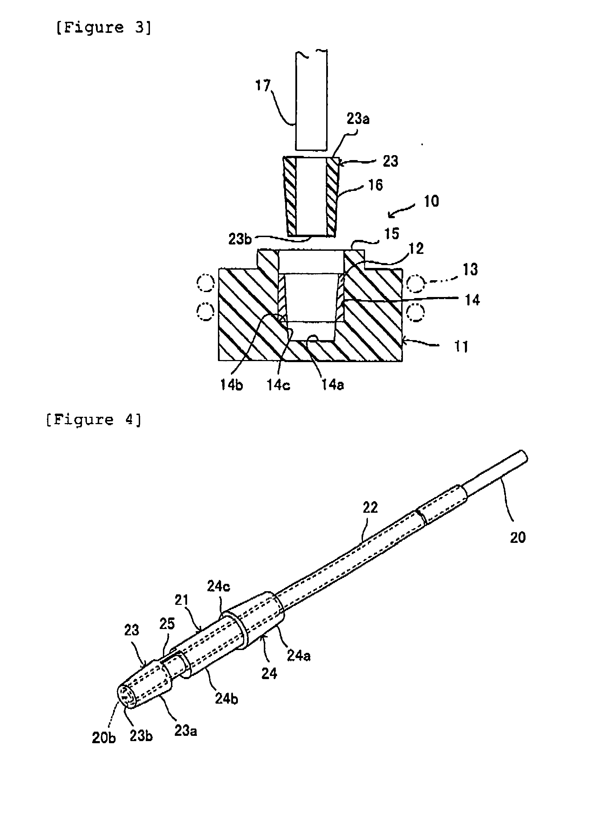

When a film 16 serving as the covering material of the present disclosure is welded to a tubular body 23a using the film welding apparatus 10 configured in this manner, the procedure is as follows. As shown in Figure 4, the tubular body 23a is a portion making up the tip end of a curve-forming member 21 for bending the tip end portion of an endoscope 20. The outer diameter of this tubular body 23a grows narrower from the base end (upper end) towards the tip end (lower end), and said tubular body has a tapering cylindrical shape (the outer shape is that of a truncated cone). Furthermore, the inner diameter of the tubular body 23a is the same in the axial direction (the vertical direction in Figure 1). The tubular body 23a is made of synthetic resin, for example polypropylene (PP), and the film 16 is made of synthetic resin, for example transparent polypropylene (PP). It should be noted that the tubular body 23a makes up part of a curve-forming member 21 comprising, as a single piece, a base body 24 and a linear linking piece 25 which links the tubular body 23a to the base body 24 (to be described later), but only the tubular body 23a is shown in Figures 1 to 3 for ease of description.

The tubular body 23a and the film 16 are first of all inserted into the heating body 12 so that the tubular body 23a is caught from the tip end opening at the outer periphery and covered by the film 16 (set-up step). That is to say, the film 16 is prepared as a tapering shape or it is left as a large film, placed horizontally between the tubular body 23a and the holding stand 11, and the tubular body 23a is inserted into the recess 14 for holding a heating body from this state (Figure 2). In this case, the holding pin 17 is inserted into the tube of the tubular body 23a so that it can be withdrawn from the base end side of the tubular body 23a. The holding pin 17 is cylindrical, and its outer diameter is substantially the same as the inner diameter of the tubular body 23a. Consequently, deformation of the tubular body 23a inside the tube is prevented. The holding pin 17 is made of a material which constitutes a non-heating body, e.g. brass, and is not heated by the induction heating. The tubular body 23a which is supported by this holding pin 17 is inserted into the recess 14 for holding a heating body so that the tip end is oriented towards the inner bottom face 14a and also the outer peripheral surface is oriented towards the inner peripheral surface of the heating body 12.

By means of this, the intermediate area between the tip end and the base end of the tubular body 23a fits tightly into the heating body 12. By inserting the tubular body 23a, the film 16 is pressured between the tip end of the tubular body 23a and the recess 14 for holding a heating body, between the outer peripheral surface of the tubular body 23a and the inner peripheral surface 14c of the recess 14 for holding a heating body, and between the outer peripheral surface of the tubular body 23a and the inner peripheral surface of the heating body 12. The film 16 deforms into the shape formed from the tip end of the tubular body 23a around the outer peripheral surface of the tubular body 23a. The tubular body 23a acts as the male mould, with the heating body 12 and the holding stand 11 together acting as the female mould for the film 16. It should be noted that the film 16 is depicted as a single line, and its thickness has been ignored.

Once the tubular body 23a and the film 16 have been set up, electrical current flows to the coil 13 to heat the heating body 12. The tip end opening of the tubular body 23a is closed off by the film 16 being welded to the outer periphery of the tubular body 23a by the heat generated by the heating body 12 (welding step). That is, the film 16 made of polypropylene (PP) is heated by the heating body 12 up to its melting temperature, and then the film 16 and the tubular body 23a are heated by means of the heated heating body 12 at the outer peripheral surface of the tubular body 23a in an overlapping state, and the film 16 is welded to the tubular body 23a.

The portion of the film 16 corresponding to the inner peripheral surface of the heating body 12 forms the welded portion. The unwelded portion of the film 16 above the welded portion extends in the opposite direction to the direction of insertion of the tubular body so as to carry on following the outer peripheral surface of the tubular body 23a from the upper end of the heating body 12, and then comes into contact with the inner peripheral edge at the upper end of the protrusion 15, opening outwards from the upper end (base end) of the tubular body 23a like a horn. If tension is applied to the portion of the film 16 extending from the welded portion in the opposite direction to the direction of insertion of the tubular body, the film 16 is fusion-cut at the upper end of the heating body 12 (at the boundary between the welded portion and the unwelded portion) because of the tension applied to the film 16 at the same time as the film 16 is welded. Moreover, it is simple to apply tension to the film 16 by bringing the film 16 into contact with the protrusion 15. By doing so, as shown in Figure 3, it is possible to obtain a leading body 23 (a tubular body with a covered tip end in which the covering material has been welded to the outer periphery thereof) having at its tip end a window part 23b corresponding to the tip end of an endoscope, which acts as a tubular body with a closed-off tip end in which the tip end opening of the tubular body 23a has been closed off. Moreover, the time required for the welding and fusion-cutting is around 1 - 2 seconds, for example. After the welding, the tubular body 23a is lifted up and taken out of the recess 14 for holding a heating body, and the holding pin 17 is withdrawn from the tubular body 23a.

Moreover, the following exemplary dimensions are possible for the tubular body 23a, film 16, heating body 12, holding stand 11 and holding pin 17. That is, for the tubular body 23a: length in the axial direction: 3.0 mm, outer diameter of tip end: 2.4 mm, outer diameter of base end: 2.5 mm, thickness: 0.2 - 0.3 mm; for the film 16: thickness: 0.03 mm; for the heating body 12: length in the axial direction 1.5 mm, outer diameter 5 mm, inner diameter 2.3 mm at the lower end and 2.4 mm at the upper end; for the holding stand 11: distance in the axial direction between the upper end of the protrusion 15 and the upper end of the heating body 12: 1 - 2 mm, distance in the axial direction between the inner bottom face 14a of the recess 14 for holding a heating body and the lower end of the heating body 12: 1 mm; and for the holding pin 17: length in the axial direction: 50 mm, outer diameter: 2.1 mm.

Furthermore, as shown in Figure 4, the curve-forming member 21 is a long, flexible, round tube which is open at both ends and is provided to fit onto the outer periphery at the tip end of an endoscope sheath 22 into which the endoscope 20 is inserted. When the endoscope sheath 22 is inserted into an insertion hole of a gastrostomy catheter (not depicted), for example, a step 24c of the base body 24 (a step between a large-diameter part 24a and a small-diameter part 24b) engages with an engaging part of the insertion hole of the gastrostomy catheter so that the base body 24 cannot proceed any further forward, but the endoscope sheath 22 moves relative to the base body 24 and can move forward. Meanwhile, the distance between the leading body 23 and the small-diameter part 24b of the base body 24 is kept constant at the linking points at both ends of the linear linking piece 25, and as the endoscope sheath 22 moves forward the tip end of the endoscope sheath 22 bends so that the head swings, and the orientation of a lens 20b (window part 23b corresponding to the tip end of an endoscope at the tip end of the leading body 23 of the curve-forming member 21) at the tip end of the endoscope 20 is changed.

The action of this mode of embodiment will be described next. The tubular body 23a and the film 16 are inserted into the heating body 12 so that the tubular body 23a is caught from the tip end opening at the outer periphery and covered by the film 16, and in this state electrical current flows to the coil 13 so that the heating body 12 is heated by induction heating. Accordingly, the film 16 is welded to the outer periphery of the tubular body 23a by the heat generated by the heating body 12, and the tip end opening of the tubular body 23a is closed off. In this case, the welding of the film 16 to the tubular body 23a takes place at the outer periphery of the tubular body 23a, and therefore the film 16 can be reliably welded to the tubular body 23a even if the tubular body 23a is thin and has an extremely small area at its end face.

In addition, the film 16 is welded to the outer periphery of the tubular body 23a, and therefore the film 16 is unlikely to become detached even if an external force in the axial direction of the tubular body 23a is applied to the film 16. Furthermore, if tension is applied to the film 16, the film 16 is fusion-cut at the boundary between the welded portion and the unwelded portion at the same time as the welding, and no burring is generated at the end of the film 16, and therefore there is no need for two-stage processing, and a pleasing external appearance can be obtained. Furthermore, the welded portion of the film is rendered smooth by the heating body during welding, and this makes wrinkling and tightening of the film unlikely to occur.

Moreover, in a state in which the tubular body 23a is inserted into the heating body 12 in the recess 14 for holding a heating body and the tip end of the tubular body 23a has reached the inner bottom face 14a of the recess 14 for holding a heating body, the outer peripheral surface of the tip end of the tubular body 23a is not facing the inner peripheral surface of the heating body 12, but is facing the inner peripheral surface 14c of the recess 14 for holding a heating body. The outer peripheral surface of the tip end of the tubular body 23a is outside the welded portion of the film 16, and therefore the outcome is that the welding has little effect on the end face at the tip end of the tubular body 23a. Furthermore, the area of the welded portion of the film 16 can be freely adjusted by changing the length in the axial direction of the heating body 12.

Furthermore, as a result of having the protrusion 15, the portion of the film 16 which is not welded to the tubular body 23a, i.e. the unwelded portion, is blocked at the cylindrical protrusion 15 so that it is unlikely to come into contact with the upper face of the heating body 12, and therefore it is possible to prevent the portion of the film 16 which is not welded to the tubular body 23a from slackening and coming into contact with the heating body 12 so that it is welded to the heating body 12. Furthermore, it is simple to apply tension to the film 16 by bringing the film 16 into contact with the protrusion 15, and this tension allows the film 16 to be fusion-cut at the end of the heating body 12 on the side in the opposite direction to the direction of insertion of the tubular body (the boundary between the welded portion and unwelded portion), which means that no burring is generated at the end of the film 16.

In addition, as a result of having the holding pin 17, the film 16 is welded to the tubular body 23a with the holding pin 17 inserted into the tubular body 23a, and deformation of the tubular body 23a inside the tube is avoided (support of the inner diameter) during welding so that it is possible to prevent wrinkling of the film and buckling of the tubular body. This is especially effective when the tubular body 23a is thin.

Furthermore with the leading body 23 of the curve-forming member 21 of the endoscope 20, the tubular body 23a is thin and it can be anticipated that external force will be applied to the film 16 in the axial direction of the tubular body 23a due to movement in the axial direction inside the body accompanying observation with the endoscope 20, but it is nonetheless effective.

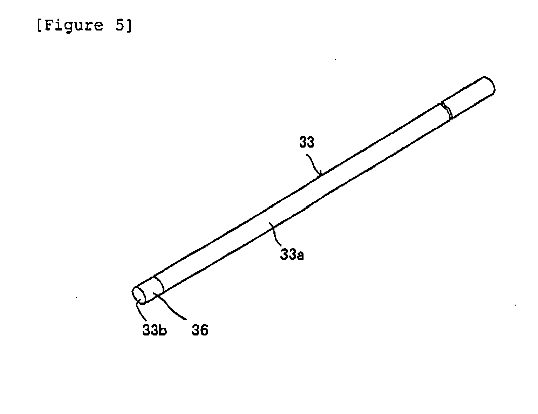

The present disclosure is not limited to the mode of embodiment described above, and various modifications can be made. For example, the tubular body 23a of the leading body 23 of the curve-forming member 21 (the tubular member for bending the endoscope) has been given as the tubular body according to the present disclosure, but this is not limiting. As shown in Figure 5, for example, the tip end opening of a tubular portion 33a of an endoscope sheath 33 may be closed off and a film 36 welded to the outer periphery of the tubular portion 33a of the endoscope sheath 33 so as to form a window part 33b corresponding to the tip end of the endoscope at the tip end thereof, rather than forming a window part corresponding to the tip end of the endoscope at the tip end of the tubular body of the leading body. In this case, the overall length of the tubular portion 33a acting as the tubular body of the endoscope sheath 33 is several tens of centimetres. Consequently, use is made of a holding pin 17 whereof the length corresponds to that of the tubular portion 33a. Furthermore, rather than providing a curve-forming member for making the endoscope bend separately from the endoscope sheath, it is possible to attach a tubular member for making the endoscope bend to the tip end of the endoscope sheath, and to weld a film onto the outer periphery of said tubular member to close off the tip end opening of the tubular member, thereby obtaining an endoscope sheath as a single piece which can bend the endoscope.

The endoscope sheath does not necessarily need to be able to cause bending of the tip end portion of the shaft of the endoscope, and it may also be a member which covers the shaft of the endoscope to prevent soiling of the shaft of the endoscope. The window part corresponding to the tip end of the endoscope need not be transparent, and it may be translucent, but higher transparency is preferable for observation. Furthermore, the tubular body is not limited to the tubular portion of the endoscope sheath. However, if it is the tubular portion of the endoscope sheath, this tubular portion is effective because it is thin. In addition, rather than providing the window part corresponding to the tip end of the endoscope at the tip end of the tubular body, the present disclosure can still be applied if a mesh-like filter is provided at the tip end of the tubular body.

Furthermore, the material and dimensions and the like, for the tubular body, film, heating body, holding stand and holding pin are not limited to those given in the mode of embodiment described above. In addition, the tubular body 23a has a tapering external shape, but this is to facilitate insertion when the endoscope sheath is inserted into the body, or to make it possible to insert the tubular body into the heating body even if there are slight variations in the dimensions of the outer diameter of the tubular body so as to obtain a good welding effect, or in view of the insertion and removal of the tubular body into the recess for holding a heating member when the film is welded and this shape is not limited to a tapering shape. Furthermore, the heating means are not limited to an induction-heating coil, and a heat source such as a heater may be used. That is to say, the heating body is not limited to a means of heating by induction heating, and it may be a means of heating from a heat source or the like, such as an external heater.

## Claims

1. Welding apparatus provided with a holding stand having a recess for holding a heating body, a cylindrical heating body which is arranged inside the abovementioned recess for holding a heating body in a state in which the outer peripheral surface is supported by the peripheral surface of the abovementioned recess for holding a heating body, and heating means for heating the abovementioned heating body,
said welding apparatus being **characterized in that** a tubular body and a covering material are inserted into the abovementioned heating body so that the abovementioned tubular body is caught from the tip end opening at the outer periphery and covered by the abovementioned covering material, and the abovementioned covering material is welded to the outer periphery of the abovementioned tubular body by the heat generated by the abovementioned heating body which is heated by means of the operation of the abovementioned heating means.

2. Welding apparatus according to Claim 1, in which a cylindrical protrusion which projects outwards is provided at the opening edge part of the abovementioned recess for holding a heating body in the abovementioned holding stand.

3. Welding apparatus according to Claim 1, in which provision is made for a holding pin for insertion into the abovementioned tubular body, the abovementioned holding pin preventing deformation of the abovementioned tubular body inside the tube by insertion of said holding pin into the abovementioned tubular body.

4. Tubular body with a covered tip end in which the abovementioned covering material is welded to the outer periphery thereof using the welding apparatus according to any one of Claims 1 to 3.

5. Welding method comprising:
a set-up step in which a tubular body and a covering material are inserted into a cylindrical heating body so that the abovementioned tubular body is caught from the tip end opening at the outer periphery and covered by the abovementioned covering material; and
a welding step in which the abovementioned heating body is heated so that the abovementioned covering material is welded to the outer periphery of the abovementioned tubular body by the heat generated by the abovementioned heating body.

6. Welding method according to Claim 5, in which the abovementioned tubular body is the tubular portion of an endoscope sheath into which an endoscope is inserted and the abovementioned covering material is a film, and the abovementioned endoscope sheath is formed by closing off the tip end opening of the abovementioned tubular body using the abovementioned film.

7. The method according to claim 5 for producing a tubular body with a covered tip end wherein a covering material is welded to the outer periphery of the tubular body, comprising:
(a) inserting a tubular body and a covering material into a cylindrical heating body,
(b) catching the tubular body from the tip end opening, at the outer periphery of the tubular body,
(c) covering the tubular body with the covering material, and
(d) heating the cylindrical heating body to generate heat to weld the covering material to the outer periphery of the tubular body.

8. The method according to claim 7 wherein the tubular body is the tubular portion of an endoscope sheath into which an endoscope is inserted, the covering material is a film, and the endoscope sheath is formed by closing off the tip end opening of the tubular body using the film.

9. The tubular body with a covered tip end in which a covering material is welded to the outer periphery of the tubular body, produced according to claim 7.

10. The endoscope sheath with a tip end opening wherein a film is welded to the outer periphery of the tubular body, produced according to claim 8.

11. The method according to claim 7 wherein the heating is carried out in a welding apparatus provided with a holding stand having a recess for holding a heating body, a cylindrical heating body which is arranged inside the recess for holding a heating body in a state in which the outer peripheral surface of the heating body is supported by the peripheral surface of the recess for holding a heating body, and heating means for heating the heating body.

12. The method according to claim 11 wherein the welding apparatus is provided with a cylindrical protrusion which projects outwards at the opening edge part of the recess for holding a heating body in the holding stand.

13. The method according to claim 11 wherein the welding apparatus is provided with a holding pin for insertion into the tubular body, the holding pin preventing deformation of the tubular body inside the tube by insertion of said holding pin into the tubular body.

14. A tubular body with a covered tip end in which a covering material is welded to the outer periphery of the tubular body, produced according to claim 11.

15. An endoscope sheath with a tip end opening wherein a film is welded to the outer periphery of the tubular body, produced according to claim 11.
